# DEMANDE DE BREVET EUROPEEN

(11) **EP 1 728 498 A1**
(43) Date de publication de la demande: **06.12.2006**
(21) Numéro de dépôt: 06113760.0
(22) Date de dépôt: 10.05.2006
(51) Int. Cl.: A61K 8/06, A61K 8/73, A61K 8/81, A61K 8/88, A61K 8/92, A61Q 1/02

(54) **Produit cosmétique bicomposition, ses utilisations, et kit de maquillage contenant ce produit.**

(30) Priorité: 01.06.2005 FR 0551451
(71) Demandeur: L'ORÉAL, 75008 Paris (FR)
(72) Inventeur: Chevalier, Véronique, 94440 Villecresnes (FR)
(74) Mandataire: Rasson, Catherine

(57) **Abrégé**

L'invention concerne un produit cosmétique de maquillage et/ou de soin de la peau, des lèvres ou des phanères contenant une première et une seconde compositions, la première composition consistant en une émulsion E/H contenant au moins une cire et des fibres, et la seconde composition comprenant une phase grasse contenant au moins une huile.

Le produit peut constituer un fond de teint, un fard à joues ou à paupières, un produit anti-cerne, un blush, un rouge à lèvres, un baume à lèvres, un brillant à lèvres, un crayon à lèvres ou à yeux, un mascara, un eye-liner, un produit de soin de la peau, un produit de maquillage du corps ou de coloration de la peau.

L'invention se rapporte également à procédé de maquillage utilisant ce produit, ainsi qu'à un kit de maquillage contenant ledit produit.

## Description

La présente invention se rapporte à un nouveau produit cosmétique comprenant au moins deux compositions qui peuvent être appliquées successivement sur la peau aussi bien du visage que du corps humain, sur les paupières inférieures et supérieures, sur les lèvres, ou sur les phanères (notamment cils) d'êtres humains.

Le produit et l'une et/ou l'autre des compositions constituant le produit peuvent être un fond de teint, un fard à joues ou à paupières, un produit anti-cerne, un blush, un rouge à lèvres, un baume à lèvres, un brillant à lèvres, un crayon à lèvres ou à yeux, un mascara, un eye-liner, un produit de soin de la peau ou un produit de maquillage du corps ou de coloration de la peau.

Dans le domaine cosmétique et notamment dans le domaine du maquillage, la mise au point de compositions dotées à la fois de bonnes propriétés en terme d'application et de confort sur la peau, et de propriétés satisfaisantes en terme de tenue, notamment de tenue de la brillance et de non migration, reste un objectif permanent. Les matières premières généralement utilisées pour la brillance sont des huiles visqueuses très brillantes à fort indice de réfraction, de préférence hydrocarbonées comme les polybutènes par exemple. Cependant, ces matières premières peuvent dans certaines conditions poser des problèmes de collant et surtout de migration lorsqu'on les utilise à fort taux.

La migration importante d'une phase grasse contenant des huiles et chargée de matières colorantes ou de produits actifs conduit à un effet inesthétique, accentuant particulièrement les rides et les ridules, ou rendant plus flou le contour du dépôt. Cette migration est souvent citée par les femmes comme un défaut majeur des rouges à lèvres classiques.

On sait qu'il est possible de limiter la migration des compositions grâce à l'addition de charges. Toutefois, on a aussi constaté que cette addition de charge s'effectuait généralement au détriment de la brillance des compositions obtenues et amenait souvent une sècheresse ressentie lors de l'application des formules.

En conséquence, il subsiste le besoin d'obtenir des compositions brillantes dotées d'une intensité de couleur et d'un confort satisfaisants sans migration.

L'invention permet de surmonter les inconvénients des produits de l'art antérieur. Elle a pour objet un produit cosmétique de maquillage et/ou de soin de la peau, des lèvres ou des phanères, contenant une première et une seconde compositions,
- la première composition consistant en une émulsion E/H comprenant une phase aqueuse dispersée dans une phase huileuse, et contenant au moins une cire et des fibres,
- la seconde composition comprenant une phase grasse contenant au moins une huile.

Grâce à l'application de la première composition, le produit de l'invention permet de limiter la migration de la deuxième composition hors du tracé ou du dépôt initial de ladite composition. La migration peut se traduire par le filage de la composition dans les rides et ridules qui sont situées à la périphérie du dépôt. La migration peut également se traduire par un débordement continu de la composition sur toute la périphérie du dépôt.

Le produit de l'invention permet par exemple de limiter la migration de la deuxième composition en dehors des lèvres ou autour des yeux.

La première composition est donc destinée à fixer le dépôt de la deuxième composition qui aurait tendance à migrer hors du tracé initial si elle était appliquée seule directement sur la peau ou les lèvres.

La demanderesse a sélectionné une première composition utilisée à titre de base pour limiter la migration d'une composition contenant une phase grasse contenant au moins une huile.

La sélection d'une base particulière permet d'obtenir un produit cosmétique, par exemple un produit de maquillage, en particulier brillant et confortable, qui ne migre pas, ce qui est difficile à obtenir.

Le produit de l'invention est particulièrement approprié pour réaliser des produits de maquillage des lèvres tels que rouges à lèvres, brillants à lèvres, des yeux tels que eye-liners, fards à paupières, ou de la peau du visage tels que les fonds de teint ou les fards à joues.

Par « produit de maquillage », on entend un produit contenant un agent colorant permettant le dépôt d'une couleur sur la peau ou les lèvres, tels que des rouges à lèvres, des fards, des eye-liners, des fonds de teint ou des autobronzants, des produits de maquillage semi permanent (tatouage).

Le produit selon l'invention comprend deux compositions conditionnées séparément ou ensemble dans un même article de conditionnement ou dans deux (ou plusieurs) articles de conditionnement séparés ou distincts. De préférence, ces compositions sont conditionnées séparément et avantageusement, dans des articles de conditionnement séparés.

L'invention a aussi pour objet un kit de maquillage contenant un produit cosmétique de maquillage ou de soin tel que défini précédemment, dans lequel les deux compositions sont conditionnées séparément et sont accompagnées de moyens d'application appropriés. Ces moyens peuvent être des pinceaux, des brosses, des stylos, des crayons, des feutres, des plumes, des éponges et /ou des mousses.

La première composition du produit selon l'invention constitue une couche de base appliquée sur la peau, les lèvres ou les phanères, et la seconde composition constitue une couche de dessus.

L'invention se rapporte aussi à un procédé de maquillage de la peau, des lèvres et/ou des phanères consistant à appliquer sur la peau, les lèvres ou les phanères successivement les première et deuxième compositions du produit cosmétique de maquillage tel que défini précédemment. La seconde composition est alors une composition de maquillage contenant des pigments ou colorants.

L'invention se rapporte aussi à un procédé de maquillage ou de soin de la peau et/ou des lèvres et/ou des phanères, consistant à appliquer sur la peau, les lèvres et/ou des phanères, une première couche de ladite première composition décrite précédemment, puis à appliquer sur cette première couche, une seconde couche de la seconde composition décrite précédemment.

La seconde couche peut former des motifs et peut être appliquée avec un stylo, crayon ou tout autre instrument (éponge, doigt, pinceau, brosse, mousse, plume etc.). Ce maquillage peut aussi être appliqué sur les accessoires de maquillage comme les faux ongles, faux cils, perruques ou encore des pastilles ou des patchs adhérents sur la peau ou les lèvres (du type mouches).

L'invention a encore pour objet l'utilisation cosmétique d'une composition cosmétique sous forme d'émulsion E/H comprenant au moins une cire et des fibres, pour diminuer la migration d'une composition cosmétique comprenant une phase grasse contenant au moins une huile.

L'invention a encore pour objet l'utilisation cosmétique pour le soin ou le maquillage des lèvres, d'une composition sous forme d'émulsion E/H comprenant au moins une cire et des fibres, pour limiter la migration d'une autre composition comprenant une phase grasse contenant au moins une huile.

### l. Première composition

La première composition constitue la base et est utilisée comme première couche. Elle se présente sous forme d'émulsion E/H comprenant une phase aqueuse dispersée dans une phase huileuse, cette dispersion étant de préférence réalisée à l'aide d'un tensioactif émulsionnant, de préférence siliconé.

Cette émulsion E/H étant destinée à une application topique, elle comprend un milieu physiologiquement acceptable. On entend par « milieu physiologiquement acceptable » un milieu compatible avec les matières kératiniques telles que la peau, les lèvres, les ongles, le cuir chevelu et/ou les cheveux.

L'émulsion E/H contient des fibres, et la phase huileuse de cette émulsion contient au moins une cire. Selon un mode préféré de réalisation de l'invention, la phase huileuse de l'émulsion E/H se présente sous forme d'une pâte souple à température ambiante.

On entend ici par « température ambiante » une température allant d'environ de 20 à 25°C.

On entend par "pâte souple", une pâte dont on peut mesurer la viscosité, par opposition à la structure solide d'un bâton ou stick, dont on ne peut pas mesurer la viscosité. La viscosité dynamique de la pâte souple à 25°C est généralement comprise entre 3 et 35 Pa.s, mesurée avec un viscosimètre rotatif CONTRAVES TV équipé d'un mobile "MS-r4" à la fréquence de 60 Hz. Habituellement, la phase huileuse qui contient des cires est préparée à chaud et, quand on la refroidit, elle se resolidifie si bien que son mélange avec la phase aqueuse doit être faite aussi à chaud pour éviter cette resolidification, alors que dans le cas où la phase huileuse se présente sous forme d'une pâte souple à température ambiante, cela signifie qu'elle ne se resolidifie pas et qu'on peut la mélanger à la phase aqueuse à température ambiante.

Cette pâte souple est susceptible d'être obtenue par exemple par chauffage des cires et autres corps gras (huiles et autres corps gras) de la phase huileuse (sauf le tensioactif émulsionnant ajouté souvent ultérieurement), puis malaxage du mélange tout en le refroidissant jusqu'à température ambiante. On peut aussi faire fondre d'abord les cires et corps gras solides, puis y ajouter les huiles et autres constituants de la phase huileuse et malaxer le mélange tout en le refroidissant, le point important pour obtenir une pâte souple étant le refroidissement au cours du malaxage.

Selon un mode plus particulièrement préféré de réalisation de l'invention, la phase huileuse et/ou l'émulsion E/H sont préparées à l'aide d'un malaxeur tel qu'un mélangeur-extrudeur.

L'émulsion E/H constituant la première composition de l'invention se présente sous forme d'une crème, c'est-à-dire d'un produit souple par opposition à un produit solide tel qu'un stick. Une crème a une viscosité à la température ambiante (environ 20 à 25°C) allant d'environ 10 à 250 poises (1 à 25 Pa.s) et de préférence allant d'environ 10 à 100 poises (1 à 10 Pa.s), cette viscosité étant mesurée avec un Rhéomat 180 avec un mobile approprié (2, 3 ou 4 par exemple).

Quand la phase huileuse de l'émulsion E/H se présente sous forme de pâte souple, l'émulsion E/H obtenue, même quand elle contient un fort taux de cire, présente l'avantage de pouvoir contenir un taux important de phase aqueuse. De ce fait, elle a l'avantage d'être fraîche à l'application sur le support topique (peau ou lèvres ou phanères). En outre, le mélange de la phase aqueuse et de la phase huileuse peut être fait à froid, et on peut ainsi incorporer des composés thermosensibles sans craindre leur dégradation.

### Fibres

Les fibres peuvent être présentes dans l'émulsion E/H de l'invention en une quantité allant de 0,1 à 30 % en poids, de préférence de 1 à 20 % en poids, mieux 3 à 15 % en poids et encore mieux de 5 à 10 % en poids par rapport au poids total de la composition.

Les fibres sont de préférence introduites dans la phase hydrophobe (ou huileuse).

Par "fibre", il faut comprendre un objet de longueur L et de diamètre D tel que L soit supérieur à D, et de préférence, très supérieur à D, D étant le diamètre du cercle dans lequel s'inscrit la section de la fibre. En particulier, le rapport UD (ou facteur de forme) est choisi dans la gamme allant de 3,5 à 2500, de préférence de 5 à 500, et mieux de 5 à 150.

Les fibres utilisables dans la composition de l'invention peuvent être des fibres d'origine synthétique ou naturelle, minérale ou organique, et elles peuvent être souples ou rigides. Elles peuvent être courtes ou longues, unitaires ou organisées par exemple tressées. Leur forme peut être quelconque et notamment de section circulaire ou polygonale (carrée, hexagonale ou octogonale) selon l'application spécifique envisagée. En particulier, leurs extrémités sont épointées et/ou polies pour éviter de se blesser.

En particulier, les fibres ont une longueur allant de 1 µm à 10 mm, de préférence de 0,1 mm à 5 mm et mieux de 0,1 mm à 3 mm. Elles ont une section comprise dans un cercle de diamètre allant de 2 nm à 500 µm, de préférence allant de 100 nm à 100 µm. Le poids des fibres est souvent donné en denier ou décitex.

Les fibres peuvent être celles utilisées dans la fabrication des textiles et notamment des fibres de soie, de coton, de laine, de lin, les fibres de cellulose extraites par exemple du bois, des légumes ou des algues, les fibres de polyamide (Nylon^{®}, notamment sous les appellations Nylon 6 = Polyamide 6 ; Nylon 6,6 ou Nylon 66 = Polyamide 6,6 ; Nylon 12 = Polyamide 12), de rayonne, de viscose, d'acétate notamment d'acétate de rayonne, d'acétate de cellulose ou d'acétate de soie, de poly-p-phénylène téréphtamide, de polymère acrylique notamment de polyméthacrylate de méthyle ou de poly-2-hydroxyéthylméthacrylate, les fibres de polyoléfine et notamment de polyéthylène ou de polypropylène, les fibres de verre, de silice, de carbone notamment sous forme graphite, de polytétrafluoroéthylène (comme le Téflon^{®}), de collagène insoluble, de polyesters, de polychlorure de vinyle ou de vinylidène, d'alcool polyvinylique, de polyacrylonitrile, de chitosane, de polyuréthane, de polyéthylène phtalate, des fibres formées d'un mélange de polymères tels que ceux mentionnés ci-avant, comme des fibres de polyamide/polyester, et les mélanges de ces fibres.

On peut aussi utiliser les fibres chirurgicales comme les fibres synthétiques résorbables préparées à partir d'acide glycolique et de caprolactone (« Monocryl » de la société Johnson & Johnson) ; les fibres synthétiques résorbables du type copolymère d'acide lactique et d'acide glycolique (« Vicryl » de la société Johnson & Johnson) ; les fibres de polyester téréphtalique (« Ethibond » de la société Johnson & Johnson) et les fils d'acier inoxydable (« Acier » de la société Johnson & Johnson).

Par ailleurs, les fibres peuvent être traités ou non en surface, enrobées ou non. Comme fibres enrobées utilisables dans l'invention, on peut citer des fibres de polyamide enrobées de sulfure de cuivre pour un effet antistatique (par exemple les fibres R-STAT de la société Rhodia) ou les fibres enrobées d'un autre polymère permettant une organisation particulière des fibres (traitement de surface spécifique) ou un traitement de surface induisant des effets de couleurs/hologrammes (fibre « Lurex » de la société Sildorex, par exemple).

Les fibres utilisables dans la composition selon l'invention sont de préférence choisies parmi les fibres de polyamide, les fibres de cellulose, les fibres de polyéthylène et leurs mélanges. Leur longueur peut aller de 0,1 à 5 mm, de préférence de 0,25 à 1,6 mm, et leur diamètre moyen peut aller de 5 à 50 µm.

Selon un mode préféré de réalisation de l'invention, les fibres sont choisies parmi les fibres de Nylon 6 (ou Polyamide 6), de Nylon 6,6 ou Nylon 66 (ou Polyamide 6,6), de Nylon 12 (ou Polyamide 12) et leurs mélanges.

En particulier, on peut utiliser les fibres de polyamide commercialisées par les Etablissements P. Bonte sous le nom Polyamide 0.9 Dtex 0.3 mm (nom INCl : Nylon 6,6), ayant un diamètre moyen de 6 µm, un poids d'environ 0.9 dtex et une longueur allant de 0,3 mm à 3 mm, ou bien encore les fibres de polyamides vendues sous la dénomination Fiberlon 931-D1-S par la société LCW, ayant un titre d'environ 0,9 dtex et une longueur d'environ 0,3 mm. On peut utiliser aussi les fibres de Nylon-66, ayant un titre d'environ 2 Dtex et une longueur d'environ 0,3 mm, commercialisées sous la dénomination « Polyamide brillante trilobée » par la société Utexbel (nom INCl : Nylon-66).

On peut aussi utiliser les fibres de celluloses (ou de rayonne) ayant un diamètre moyen de 50 µm et une longueur allant de 0,5 mm à 6 mm comme celles vendues sous le nom de "Natural rayon flock fiber RC1BE - N003 - M04" par la société Claremont Flock. On peut également utiliser des fibres de polyéthylène comme celles vendues sous le nom de "Shurt Stuff 13 099 F" par la société Mini Fibers.

### Cires

L'émulsion E/H de l'invention contient une ou plusieurs cires présentes dans la phase huileuse. La quantité de cire(s) dans l'émulsion E/H de l'invention est de préférence d'au moins 4 % en poids et mieux d'au moins 5 % en poids par rapport au poids total de l'émulsion. Elle peut aller par exemple de 4 à 30 %, de préférence de 5 à 30 % en poids et mieux de 5 à 15 % en poids par rapport au poids total de l'émulsion.

Comme cires utilisables dans l'émulsion E/H de l'invention, on peut citer par exemple les cires minérales telles que les cires microcristallines, la paraffine, le pétrolatum, la vaseline, l'ozokérite, la cire de montan ; les cires d'origine animale telles que la cire d'abeilles, la lanoline et ses dérivés ; les cires d'origine végétale telles que les cires de Candellila, d'Ouricurry, de Carnauba, du Japon, le beurre de cacao, les cires de fibres de lièges ou de canne à sucre ; les huiles hydrogénées concrètes à 25°C ; les esters gras et les glycérides concrets à 25°C ; les cires synthétiques telles que les cires de polyéthylène et les cires obtenues par synthèse de Fischer-Tropsch ; les cires de silicone, et leurs mélanges.

Selon un mode préféré de réalisation de l'invention, on utilise au moins une cire ayant une température de fusion commençante supérieure ou égale à 50°C, et mieux au moins une cire dont la température de fusion commençante est supérieure ou égale à 65°C, telles que la cire de Carnauba, les cires de polyéthylène ayant une température de fusion commençante supérieure à 50°C et mieux à 65°C et les cires microcristallines ayant une température de fusion commençante supérieure à 50°C et mieux 65°C, telles que celle vendue par la société Tisco sous la dénomination Tisco Wax 88 ou celle vendue par la société RMC sous la dénomination Feruwax 30540, et leurs mélanges.

Par "température de fusion commençante", on entend dans la présente description, la température à laquelle une cire commence à fondre. On peut déterminer cette température par ATD (analyse thermique différentielle) qui permet l'obtention du thermogramme (ou courbe de fusion) de la cire considérée. La température de fusion commençante correspond à la température à laquelle on peut observer un changement de pente notable dans le thermogramme. Le point de fusion, quant à lui, représente le point minimum dudit thermogramme.

### Tensioactif émulsionnant

Selon un mode préféré de réalisation de l'invention, l'émulsion E/H contient au moins un tensioactif émulsionnant. Ce tensioactif est de préférence un tensioactif siliconé. La quantité (en matière active) de tensioactif(s) émulsionnant(s) dans l'émulsion E/H va de préférence de 0,02 à 5 % en poids de matière active, mieux de 0,05 à 4 % en poids et encore mieux de 0,1 à 2 % en poids de matière active par rapport au poids total de la composition.

Le tensioactif émulsionnant siliconé.peut être notamment choisi dans le groupe comprenant les polydiméthylsiloxanes oxyéthylénés et/ou oxypropylénés appelés aussi dimethicone copolyols, les alkyl-C₁₀-C₂₂-polydiméthylsiloxanes oxyéthylénés et/ou oxypropylénés appelés aussi alkyldiméthicone copolyols, les polydimethylsiloxanes oxyéthylénés et/ou oxypropylénés à groupements glucosides, et leurs mélanges.

Comme dimethicone copolyols, on peut citer par exemple le mélange de dimethicone copolyol, de cyclopentasiloxane (cyclométhicone) et d'eau (10/88/2), commercialisé par la société Dow Corning sous la dénomination DC3225C ou DC-5225C (nom INCl: Cyclopentasiloxane / PEG/PPG-18/18 Dimethicone), et le mélange de dimethicone copolyol et de cyclopentasiloxane (85/15) commercialisé sous la dénomination Abil EM-97 par la société Goldschmidt (nom INCl : BIS-PEG/PPG-14/14 Dimethicone / Cyclopentasiloxane).

Comme alkyldiméthicone copolyols, on peut citer notamment ceux ayant un radical alkyle comportant de 10 à 22 atomes de carbone, tel que le cétyl diméthicone copolyol comme le produit commercialisé sous la dénomination Abil EM-90 par la société Goldschmidt ; le lauryl diméthicone copolyol et par exemple le mélange d'environ 91 % de lauryl diméthicone copolyol et d'environ 9 % d'alcool isostéarylique, commercialisé sous la dénomination Q2-5200 par la société Dow Corning, et les mélanges de ces tensioactifs siliconés.

Comme polydiméthylsiloxane à groupements glucosides, on peut citer notamment les composés de formule (I) 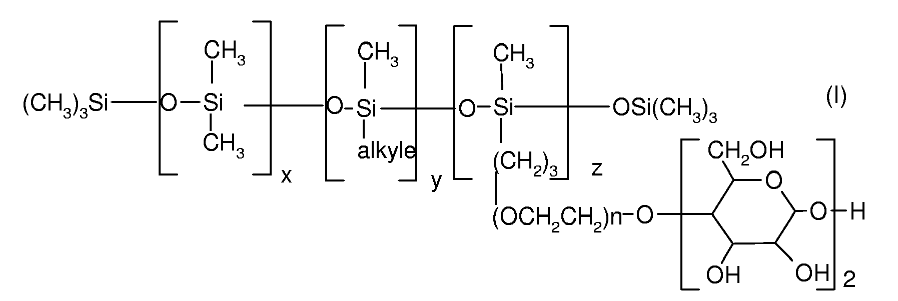 dans laquelle :
x, y et z représentent chacun un nombre entier positif allant de 1 à 10000, de préférence de 1 à 5000, et mieux de 1 à 2000,
n représente un nombre entier positif allant de 1 à 20, de préférence de 1 à 15, le radical alkyle représente un radical hydrocarboné saturé, linéaire ou ramifié, comportant de 2 à 20, et de préférence de 2 à 8 atomes de carbone,
et particulièrement le composé de formule (I) où le radical alkyle est un radical n-octyle, commercialisé sous forme d'une solution liquide à 15 % en matière active dans une cyclométhicone sous la dénomination SPG 128 VP par la société Wacker.

Le tensioactif siliconé est de préférence un diméthicone copoyol, plus particulièrement celui commercialisé sous les dénominations DC3225C ou DC5225C.

### Phase huileuse

La phase huileuse de l'émulsion E/H de l'invention peut renfermer, outre les cires indiquées ci-dessus, toute sorte d'huiles et de corps gras bien connus de l'homme du métier, comme exemple les huiles d'origine végétale (par exemple huiles de jojoba, avocat, sésame, tournesol, maïs, soja, carthame, pépins de raisin), les huiles minérales (comme vaseline, isoparaffines éventuellement hydrogénées), les huiles de synthèse (par exemple myristate d'isopropyle, octanoate de cétéaryle, polyisobutylène, palmitate d'éthyl-hexyle, alkyl-benzoates), les huiles de silicone volatiles ou non volatiles, et les huiles fluorées ou fluorosiliconées, ainsi que les mélanges de ces huiles.

De préférence, la phase huileuse de la composition de l'invention comprend au moins une huile de silicone qui peut être présente en une quantité allant par exemple de 3 à 50 % en poids et de préférence de 5 à 30 % en poids et mieux de 9 à 30 % en poids par rapport au poids total de la composition. Comme huile de silicone, on peut citer par exemple les huiles de silicone volatiles telles que les cyclodiméthylsiloxanes ou cyclométhicones, comme la pentacyclométhicone, la tétracyclométhicone ou l'hexacyclométhicone ; les huiles de silicone non volatiles telles que les polydiméthylsiloxanes (PDMS). L'huile de silicone peut être celle contenue dans la matière première constituant le tensioactif siliconé, comme c'est le cas quand on utilise le DC-5225C ou DC-3225C.

La phase huileuse peut contenir, en outre, d'autres constituants gras tels que les alcools gras comme l'alcool stéarylique, l'alcool cétylique et l'alcool cétéarylique, les acides gras, les gommes, par exemple les gommes de silicone comme le mélange PDMS à groupements alpha oméga hydroxylés / PDMS 5 cst (12/88) vendu sous la dénomination DC 1503 par la société Dow Corning, et les gélifiants lipophiles tels que la bentone.

La phase huileuse peut contenir aussi des adjuvants lipophiles.

La phase huileuse peut être présente dans l'émulsion en une quantité allant de 15 à 70 % en poids et de préférence de 25 à 60 % en poids par rapport au poids total de l'émulsion, cette quantité comprenant la quantité de tensioactif émulsionnant.

Comme indiqué ci-dessus, la phase huileuse refroidie se présente de façon avantageuse, avant son mélange avec la phase aqueuse, sous forme d'une pâte souple, c'est-à-dire ayant une viscosité dynamique de la pâte souple à 25°C généralement comprise entre 3 et 35 Pa.s, mesurée avec un viscosimètre rotatif CONTRAVES TV équipé d'un mobile "MS-r4" à la fréquence de 60 Hz.

On peut obtenir cette pâte souple notamment en chauffant les cires et autres corps solides de la phase huileuse, puis en malaxant le mélange de ce pré-mélange fondu avec les autres constituants de la phase huileuse tout en refroidissant le mélange jusqu'à température ambiante.

### Phase aqueuse

La phase aqueuse de l'émulsion E/H de l'invention peut aller de 30 à 85 % en poids et de préférence de 40 à 75 % en poids par rapport au poids total de l'émulsion. Elle contient de l'eau, et peut contenir, outre l'eau, des solvants et des adjuvants hydrophiles. Comme solvants, on peut citer par exemple les alcools primaires comportant de 1 à 4 atomes de carbone comme l'éthanol, ou les polyols comme le butylène glycol, la glycérine et l'hexylène glycol. Le ou les solvants peuvent être présents en une quantité allant de 0,1 à 30 % en poids par rapport au poids total de l'émulsion.

L'émulsion E/H peut comprendre en outre un ou plusieurs adjuvants comme décrit ci-après.

Un mode particulier de préparation de l'émulsion E/H de l'invention, consiste à effectuer la préparation de la phase huileuse et éventuellement de toute d'émulsion, à l'aide d'un mélangeur-extrudeur à vis, soumis à un gradient de température allant de 100°C à 10°C et à introduire les fibres en premier dans le mélangeur-extrudeur.

La pâte souple est susceptible d'être obtenue par exemple par fusion des cires et autres corps gras solides et malaxage de ce pré-mélange fondu avec les huiles et les autres constituants de la phase huileuse, malaxage réalisé tout en refroidissant ce mélange jusqu'à température ambiante, le malaxage pouvant notamment se faire dans un mélangeur-extrudeur.

De manière plus précise, le procédé de préparation de l'émulsion dans un mélangeur-extrudeur peut comprendre les étapes suivantes :
- (1) préparation de la phase huileuse par mélange à chaud des cires, huiles et autres constituants lipophiles de la phase huileuse, entre 80°C et 100°C,
- (2) introduction des charges et fibres dans le premier fourreau du mélangeur-extrudeur ;
- (3) introduction du mélange fondu obtenu en (1) dans le second fourreau chauffé à la température à laquelle le mélange de (1) a été préparé ;
- (4) malaxage du mélange de (2) et (3) tout en le refroidissant jusqu'à température ambiante par passage dans plusieurs fourreaux et obtention d'une pâte souple ;
- (5) incorporation du tensioactif émulsionnant dans la pâte souple obtenue en (4), et
- (6) incorporation, sous agitation, de la phase aqueuse et éventuellement de charges non introduites en (2) dans le mélange obtenu en (5).

Un autre procédé consiste à introduire dans le même fourreau, le mélange fondu des cires et huiles et les charges et fibres.

L'utilisation d'un mélangeur-extrudeur permet d'obtenir de façon reproductible une pâte de phase huileuse de qualité très constante. De plus, il est possible, en adaptant la filière de sortie du mélangeur-extrudeur, de conditionner l'émulsion E/H en ligne à la sortie dudit mélangeur-extrudeur.

Les différents étapes du procédé peuvent être réalisées dans un ou plusieurs extrudeurs disposés à la suite les uns des autres, et de préférence dans un extrudeur bivis unique.

Les conditions dans lesquelles l'extrusion peut être effectuée sont décrites dans le document FR-A-2,715,306 dont le contenu est incorporé à la présente demande par référence.

La longueur des fourreaux peut varier. De manière préférée, elle est de 100 mm, et on peut ajouter des fourreaux supplémentaires si nécessaire pour l'introduction séparée d'autres composés.

### II. Seconde composition

La seconde composition comprend une phase grasse contenant au moins une huile. Cette composition constitue la couche supérieure du produit et s'applique au-dessus de la base constituée par la première composition.

Par « huile », au sens de la demande, on entend tout milieu non aqueux liquide à température ambiante (20-25°C) et à pression atmosphérique (760 mm de Hg).

La deuxième composition peut n'être constituée que de la phase grasse. Elle peut aussi comprendre une autre phase et par exemple une phase aqueuse.

La phase grasse comprend les huiles et les autres corps gras tels que cires, gommes et corps pâteux.

La quantité d'huiles dans la deuxième composition représente de préférence au moins 20 % en poids et mieux au moins 30 % en poids du poids total de cette composition. Cette quantité peut aller par exemple de 20 à 90 % et mieux de préférence de 30 à 85 % du poids total de cette composition.

Selon un mode préféré de mise en oeuvre de l'invention, la seconde composition contient au moins une huile hydrocarbonée non volatile.

Comme huile non volatile hydrocarbonée utilisable dans l'invention, on peut citer: :
- les hydrocarbures linéaires ou ramifiés tels que les huiles de paraffine, de vaseline et de naphtalène légères, le polyisobutène hydrogéné (Huile de Parleam)
- les huiles hydrocarbonées d'origine animale comme le squalène et le perhydrosqualène (ou squalane) ;
- les triglycérides liquides d'acides gras comportant au moins 10 atomes de carbone;
- les esters et les éthers de synthèse notamment d'acides gras comme les huiles de formule R₁(CO)_{X}OR₂ dans laquelle R₁ représente le reste d'un acide comportant de 2 à 29 atomes de carbone avec x valant 0 ou 1 et R₂ représente une chaîne hydrocarbonée contenant de 3 à 30 atomes de carbone comme par exemple l'acétylcitrate de tributyle, l'érucate d'oléyle, le béhénate d'octyl-2 dodécyle, le citrate de tri-iso-arachidyle, le stéaroyl-stéarate d'isocétyle ou d'octyldodécanyle, l'acétate de n-propyle, le tri-méllitate de tri-décyle, le dodécane di-oléate ou le stéarate de di-isocétyle, le propionate d'arachidyle, le dibutyl phtalate, le carbonate de propylène, le pentanoate d'octyldodécyle ; les esters de polyol comme la vitamine F, l'isostéarate de sorbitane, le triisostéarate de glycérine ou de diglycérine , le polyglyceryl-2 diisostéarate ; le malate de diisostéaryle, le palmitate d'isopropyle, l'adipate de diisopropyle, les triglycérides des acides caprylique/caprique comme ceux vendus par la société Stearineries Dubois ou ceux vendus sous les dénominations Miglyol 810, 812 et 818 par la société Dynamit Nobel, l'huile de beurre de karité, le myristate d'isopropyle, le stéarate de butyle, le laurate d'hexyle, l'adipate de diisopropyle, l'isononate d'isononyle, le laurate de 2-hexyl-décyle, le palmitate de 2-octyl-décyle, le myristate ou le lactate de 2-octyl-dodécyle, le succinate de 2-diéthyl-hexyle, le palmitate d'éthyl-2-hexyle, le stéarate d'octyl-2-dodécyle, l'huile de ricin ; le dimérate de di-isopropyle (nom INCI : Diisopropyl Dimer Dilinoleate) tel que le produit commercialisé sous la dénomination SCHERCEMOL DID par la société Noveon ;
- les huiles fluorées;
- les alcools gras comportant de 7 à 29 atomes de carbone, tels que l'alcool stéarylique, l'alcool linoléique ou linolénique, l'alcool isostéarique, l'octyl 2-dodécanol, le décanol, le dodécanol, l'octadécanol ou l'alcool oléique ;
- les acides gras comportant de 7 à 29 atomes de carbone, tels que l'acide myristique, l'acide palmitique, l'acide stéarique, l'acide béhénique, l'acide oléique, l'acide linoléique, l'acide linolénique ou l'acide isostéarique ;
- leurs mélanges.

La seconde composition peut contenir aussi des huiles de silicone, de préférence des huiles de silicone non volatiles. Comme huiles de silicone non volatiles, on peut citer les polydiméthylsiloxanes (PDMS), comportant éventuellement une chaîne alkyle ou alcoxy en C₃-C₄₀ ou une chaîne phénylée telle que les phényltriméthicones, les polyalkylméthylsiloxanes, éventuellement fluorés comme les polyméthyltrifluoro-propyldiméthylsiloxanes, ou par des groupements fonctionnels tels que des groupements hydroxyle, thiol et/ou amine; les polysiloxanes modifiés par des acides gras, des alcools gras ou des polyoxyalkylènes.

La deuxième composition peut comprendre aussi une ou plusieurs cires et/ou un ou plusieurs pâteux, en particulier lorsqu'elle se présente sous la forme d'un stick.

Par "pâteux" au sens de la présente invention, on entend un composé gras lipophile à changement d'état solide/liquide réversible, présentant à l'état solide une organisation cristalline anisotrope, et comportant à la température de 23°C une fraction liquide et une fraction solide.

Le composé pâteux est avantageusement choisi parmi :
- la lanoline et ses dérivés
- les composés siliconés polymères ou non
- les composés fluorés polymères ou non
- les polymères vinyliques, notamment :
   - les homopolymères d'oléfines,
   - les copolymères d'oléfines tels que les copolymères polyvinylpyrrolidone / alpha-oléfines comme le copolymère de vinylpyrrolidone et d'hexadécène commercialisé sous la dénomination Antaron V216 par la société ISP,
   - les homopolymères et copolymères de diènes hydrogénés,
   - les oligomères linéaires ou ramifiés, homo ou copolymères de (méth)acrylates d'alkyles ayant de préférence un groupement alkyle en C₈₋C₃₀,
   - les oligomères homo et copolymères d'esters vinyliques ayant des groupements alkyles en C₈-C₃₀,
   - les oligomères homo et copolymères de vinyléthers ayant des groupements alkyles en C₈-C₃₀,
- les polyéthers liposolubles résultant de la polyéthérification entre un ou plusieurs diols en C2-C100, de préférence en C2-C50,
- les esters comme le mélange d'esters d'acides gras isostéarique /adipique de glycéryle,
et leurs mélanges.

Le composé pâteux peut représenter par exemple de 1 à 99%, de préférence de 1 à 60%, mieux de 2 à 30% et mieux encore de 5 à 15% en poids de la composition constituant la seconde composition du produit selon l'invention.

Les cires peuvent être les mêmes que celles utilisées pour la première composition. Parmi les cires, on peut notamment citer les cires d'origines animale, végétale, minérale ou synthétique telles que la cire liquide de jojoba, les cires microcristallines, la paraffine, la vaseline, l'ozokérite, la cire de Montan; la cire d'abeilles, la lanoline et ses dérivés; les cires de Candellila, d'Ouricury, de Carnauba, du Japon, le beurre de cacao, les cires de fibres de lièges ou de canne à sucre; les huiles hydrogénées concrètes à 25 °C, les ozokérites, les esters gras et les glycérides concrets à 25 °C; les cires de polyéthylène et les cires obtenues par synthèse de Fischer-Tropsch; des huiles hydrogénées concrètes à 25 °C; des lanolines; des esters gras concrets à 25 °C; les cires de silicone; les cires fluorées; leurs mélanges.

La seconde composition du produit selon l'invention peut se présenter sous toute forme galénique normalement utilisée pour une application topique et notamment sous forme d'une solution huileuse ou aqueuse, d'un gel huileux ou aqueux, d'une émulsion huile-dans-eau ou eau-dans-huile, d'une émulsion multiple, d'une dispersion d'huile dans de l'eau grâce à des vésicules, les vésicules étant situés à l'interface huile/eau, ou d'une poudre. Elle peut être fluide ou solide. Elle peut avoir l'aspect d'une lotion, d'une crème, d'une pommade, d'une pâte souple, d'un onguent, d'un solide coulé ou moulé et notamment en stick ou en coupelle, ou encore de solide compacté.

Selon un mode préféré de réalisation de l'invention, elle se présente sous forme d'un solide coulé ou moulé, notamment sous la forme d'un rouge à lèvres en stick, d'un brillant à lèvres, d'un fond de teint coulé ou d'un blush coulé. Ce peut être aussi par exemple un brillant à lèvres liquide.

Avantageusement, la seconde composition est à phase grasse continue et de préférence sous forme anhydre. Le terme « anhydre » signifie que la composition peut contenir moins de 5% d'eau, et encore mieux moins de 1% d'eau par rapport au poids total de la seconde composition.

La seconde composition peut être préparée de manière usuelle par l'homme du métier. Elle peut être obtenue notamment par chauffage des différents constituants à la température de fusion des cires les plus élevées, puis coulage du mélange fondu dans un moule (coupelle ou doigt de gant). Elle peut aussi être obtenue par extrusion comme décrit dans la demande EP-A-0667146.

### Adjuvants

De façon connue, les deux compositions de l'invention peuvent contenir également des adjuvants habituels dans les domaines cosmétique et/ou dermatologique, tels que les gélifiants lipophiles ou hydrophiles, les actifs, les conservateurs, les antioxydants, les agents complexants, les ajusteurs de pH (acides ou basiques), les parfums, les charges, les bactéricides, les absorbeurs d'odeur, les colorants et encore les vésicules lipidiques. Les quantités de ces différents adjuvants sont celles classiquement utilisées dans le domaine considéré, et par exemple de 0,01 à 20% du poids total de la composition. Ces adjuvants, selon leur nature, peuvent être introduits dans la phase grasse, dans la phase aqueuse et/ou dans les vésicules lipidiques.

Ainsi, les compositions de l'invention peuvent contenir notamment des gélifiants lipophiles tels que les bentones et les hectorites, et tels que les organopolysiloxanes élastomères comme par exemple ceux commercialisés sous les noms KSG 6 de Shin-Etsu, Trefil E-505C ou Trefil E-506C de Dow-Corning, Gransil (SR-CYC, SR DMF10, SR-DC556) de Grant Industries, ou ceux commercialisés sous forme de gels déjà constitués : KSG 15, KSG 17, KSG 16, KSG 18 de Shin-Etsu, Gransil SR 5CYC gel, Gransil SR DMF 10 gel, Gransil SR DC556 gel de Grant Industries, 1229-02-167 et 1229-02-168 de General Electric. On peut aussi utiliser un mélange de ces composés.

Les charges qui peuvent être utilisées dans les compositions de l'invention, on peut citer par exemple, les matières colorantes pulvérulentes ; la poudre de silice ; le talc ; le mica ; le kaolin ; les particules de polyamide et notamment celles vendues sous la dénomination ORGASOL par la société Atochem ; les poudres de polyéthylène ; les microsphères à base de copolymères acryliques, telles que celles en copolymère diméthacrylate d'éthylène glycol/ methacrylate de lauryle vendues par la société Dow Corning sous la dénomination de POLYTRAP ; les poudres expansées telles que les microsphères creuses et notamment, les microsphères commercialisées sous la dénomination EXPANCEL par la société Kemanord Plast ou sous la dénomination MICROPEARL F 80 ED par la société Matsumoto ; les poudres de matériaux organiques naturels tels que les amidons de maïs, de blé ou de riz, réticulés ou non, telles que les poudres d'amidon réticulé par l'anhydride octénylsuccinate, commercialisées sous la dénomination DRY-FLO par la société National Starch ; les microbilles de résine de silicone telles que celles commercialisées sous la dénomination TOSPEARL par la société Toshiba Silicone ; et leurs mélanges. Ces charges peuvent être présentes dans des quantités allant de 0 à 20 % en poids et de préférence de 1 à 10 % en poids par rapport au poids total de l'émulsion.

Comme matières colorantes pulvérulentes, on peut citer en particulier les pigments, les nacres, les paillettes et leurs mélanges.

Les pigments peuvent être blancs ou colorés, minéraux et/ou organiques, interférentiels ou non. On peut citer, parmi les pigments minéraux, le dioxyde de titane, éventuellement traité en surface, les oxydes de zirconium ou de cérium, ainsi que les oxydes de zinc, de fer (noir, jaune ou rouge ou de chrome, le violet de manganèse, le bleu outremer, l'hydrate de chrome et le bleu ferrique. Parmi les pigments organiques, on peut citer le noir de carbone, les pigments de type laques organiques de baryum, strontium, calcium ou aluminium dont celles soumises à une certification par la Food and Drug Administration (FDA) (exemple D&C ou FD&C) et ceux exempts de la certification FDA comme les laques à base de carmin de cochenille. Les pigments peuvent représenter de 0,1 à 50 % en matière active et notamment de 0,5 à 35 % et mieux de 2 à 25 % du poids total de la composition.

Les pigments nacrés peuvent être choisis parmi les pigments nacrés blancs tels que le mica recouvert de titane, ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth. Ils peuvent représenter de 0 à 25 % (en matière active) du poids total de la composition et mieux de 0,1 à 15 % (si présents). On peut ainsi utiliser des pigments à propriétés goniochromatiques, et/ou des pigments à effet métallique tel que décrit dans la demande déposée pour le numéro FR-A-2,842,417 dont le contenu est incorporé par référence dans la présente demande.

Selon un mode préféré de mise en oeuvre de l'invention, la première composition contient moins de 10 % en poids de pigments, de préférence moins de 5%, de préférence encore moins de 2%, de préférence encore moins de 1% en poids de pigments, tandis que la deuxième composition contient au moins 0,1% de pigments, de préférence au moins 1% de pigments, de préférence encore au moins 5% en poids de pigments, de préférence encore au moins 10% en poids de pigments.

Comme actifs utilisables dans les compositions de l'invention, on peut citer par exemple, les enzymes (par exemple lactoperoxydase, lipase, protéase, phospholipase, cellulases) ; les flavonoïdes ; les agents hydratants tels que les hydrolysats de protéines ; le hyaluronate de sodium ; les polyols comme la glycérine, les glycols comme les polyéthylène glycols, et les dérivés de sucre ; les anti-inflammatoires ; les oligomères procyannidoliques ; les vitamines comme la vitamine A (rétinol), la vitamine E (tocophérol), la vitamine C (acide ascorbique), la vitamine B5 (panthénol), la vitamine B3 (niacinamide), les dérivés de ces vitamines (notamment esters) et leurs mélanges ; l'urée ; la caféine ; les dépigmentants tels que l'acide kojique, l'hydroquinone et l'acide caféique ; l'acide salicylique et ses dérivés ; les alpha-hydroxyacides tels que l'acide lactique et l'acide glycolique et leurs dérivés ; les rétinoïdes tels que les caroténoïdes et les dérivés de vitamine A ; les filtres solaires ; l'hydrocortisone ; la mélatonine ; les extraits d'algues, de champignons, de végétaux, de levures, de bactéries ; les stéroïdes ; les actifs anti-bactériens comme le 2,4,4'-trichloro-2'-hydroxy diphényl éther (ou triclosan), le 3,4,4'-trichlorocarbanilide (ou triclocarban) et les acides indiqués ci-dessus et notamment l'acide salicylique et ses dérivés ; les agents matifiants ; les agents tenseurs ; les céramides ; les huiles essentielles ; et leurs mélanges ; et tout actif approprié pour le but final de la composition.

Le ou les actifs peuvent être par exemple présents en une concentration allant de 0,01 à 20 %, de préférence de 0,1 à 5 % et mieux de 0,5 à 3 % du poids total de la composition.

Bien entendu, l'homme de l'art veillera à choisir le ou les éventuels composés à ajouter aux compositions de l'invention de manière telle que les propriétés avantageuses attachées intrinsèquement au produit conforme à l'invention ne soient pas ou substantiellement pas, altérées par l'addition envisagée.

### III. Produit selon l'invention

Ce produit comprend les deux compositions décrites ci-dessus. Le produit selon l'invention peut être avantageusement utilisé pour le maquillage ou le soin de la peau et/ou des lèvres et/ou des phanères selon la nature des ingrédients utilisés. En particulier, il peut constituer un fond de teint, un fard à joues ou à paupières, un produit anti-cerne, un blush, un rouge à lèvres, un baume à lèvres, un brillant à lèvres, un crayon à lèvres ou à yeux, un mascara, un eye-liner, un produit de soin de la peau, un produit de maquillage du corps ou de coloration de la peau. Le produit est en particulier un rouge à lèvres.

Chaque composition peut être conditionnée séparément dans un même article de conditionnement par exemple dans un stylo bi-compartimenté, la composition de base étant délivrée par une extrémité du stylo et la composition du dessus étant délivrée par l'autre extrémité du stylo, chaque extrémité étant fermée notamment de façon étanche par un capuchon.

II est possible aussi que chaque composition soit présentée séparément, par exemple dans deux tubes ou deux pots ou un tube et un pot.

La composition qui est appliquée en première couche est sous forme de crème, ce qui permet une application plus pratique.

Avantageusement, la couche du dessus (donnée par la deuxième composition) présente des propriétés de soin et/ou de brillance et/ou de coloration.

L'invention a encore pour objet un produit à lèvres, un fond de teint, un tatouage, un fard à joues ou à paupières, contenant une première et une seconde compositions telles que décrites précédemment.

L'exemple ci-après de compositions selon l'invention est donné à titre d'illustration et sans caractère limitatif. Les noms y sont donnés en noms chimiques, INCI ou commerciaux. Les quantités y sont données en % en poids, sauf mention contraire.

### Exemple 1

### l. Base à titre de première composition : Emulsion E/H

### A. Phase huileuse

| | |
|---|---|
| - Dry-Flo (charge) | 7 % |
| - Cire microcristalline | 19 % |
| - Fibres de polyamide (Nylon 6,6) | 19 % |
| - Huile minérale | qsp 100 % |

### Emulsion E/H

| | |
|---|---|
| - Phase huileuse | 40 % |
| - Cyclopentasiloxane / PEG/PPG-18/18 Dimethicone (DC-5225C) | 10 % |
| - Silice (charge) | 4 % |
| - Glycérine | 5 % |
| - Eau | qsp 100 % |

### Mode opératoire 1 :

- On chauffe le mélange de cire et d'huile à environ 100°C,
- on introduit ce mélange fondu dans un mélangeur-extrudeur au même niveau que le mélange de charge (Dry-Flo) et huile minérale, et les fibres sont introduites via un doseur pondéral. On obtient à la sortie du mélangeur-extrudeur, la phase huileuse sous forme d'une pâte souple,
- Dans un rotor stator, on incorpore dans la pâte souple, l'émulsionnant siliconé,
- on ajoute au mélange peu à peu l'eau, la glycérine et la silice tout en agitant.

### Mode opératoire 2:

- On chauffe le mélange de cire et d'huile à environ 100°C,
- on introduit la charge (Dry-Flo) et les fibres dans le premier fourreau d'un mélangeur-extrudeur comportant six fourreaux,
- on introduit le mélange fondu de cire et d'huile dans le second élément dudit mélangeur-extrudeur à vis, et
- on introduit la phase aqueuse, l'émulsionnant siliconé et la silice par deux entrées différentes, dans le quatrième élément dudit mélangeur-extrudeur à vis.

Les éléments du mélangeur-extrudeur bi vis utilisé sont, en allant du premier au sixième élément, portés respectivement aux températures suivantes : 100°C, 80°C, 60 °C, 20 °C, 20°C et 20 °C.

L'émulsion obtenue est crémeuse, fluide et non grasse, et elle a un fini poudré. Elle constitue une bonne base.

### II. Deuxième composition : composition anhydre

| | | |
|---|---|---|
| Cire liquide de jojoba | | 5,7 % |
| Carbonate de propylène | | 0,03 % |
| Beurre de karité | | 3, 6 % |
| Copolymère vinylpyrrolidone / hexadécène (ANTARON V 216) | | 2,4 % |
| Cire de polyéthylène (P.M.: 500) | | 6,6 % |
| Di-mérate de diisopropyle | | |
| (nom INCI : Diisopropyl dimer dilinoleate) | Qsp | 100 % |
| Esters d'acides gras,isostéarique/adipique de glycéryle | | 6,1 % |
| Mica-oxyde de titane | | 2,2 % |
| Sel de calcium du rouge lithol | | 2,5 % |
| Isoparaffine hydrogénée | | 7,1 % |
| Oxyde de fer noir | | 0,1 % |
| Oxydes de fer brun,jaune | | 1,9 % |
| Hectorite modifiée distearyl dimethyl ammonium | | 0,5 % |
| Ditertiobutyl 4-hydroxytoluène | | 0,03 % |
| Perhydrosqualène | | 5,7 % |
| Mélange de p-hydroxybenzoates d'iso-propyle,iso-butyle, | | |
| n-butyle (40/30/30) | | 0,5 % |
| Ozokerite (cire de point de fusion: 74-77°C) | | 4,9 % |
| Polyisobutène hydrogéné (huile de Parléam) | | 7,3 % |
| Laque d'aluminium | | 5 % |
| Polyglyceryl-2 diisostéarate | | 9,7 % |
| Octyl-2 dodecanol | | 6,3 % |

### Exemple 2 :

II est identique à l'exemple 1 sauf que la composition de la base (émulsion E/H) contient en outre 2 % de bentone (gélifiant huileux). Dans le mode opératoire, la bentone est introduite dans le mélange de cire, huile et Dry-Flo.

La composition a de bonnes qualités émollientes.

### Tests d'efficacité comme base de rouge à lèvres (RAL)

### 1) Flash Esthétique

Objectif : évaluer l'influence de la composition de base de l'exemple 1 sur le lissant et l'anti-filant d'un rouge à lèvres (RAL) classique sur 8 femmes ayant des ridules au contour des lèvres. On applique d'abord comme composition de base, l'émulsion E/H utilisée selon l'invention, puis un rouge à lèvres reconnu comme particulièrement filant.

Résultat : 7 utilisatrices sur 8 ont trouvé que la composition de base selon l'invention avait une très bonne performance sur la tenue du RAL, notamment du fait de son effet anti-migration et de son effet sur le maintien du film (couvrance et brillance).

### 2) Screening instrumental classique

Objectif : évaluer la tenue de la couleur, la brillance et le degré de migration en utilisant comme composition de base, l'émulsion E/H de l'exemple 1, associée au rouge à lèvres classique sur 6 femmes.

Résultat : dans les conditions expérimentales consistant à appliquer la composition selon l'invention comme base puis un RAL classique, il n'a pas été observé de migration du rouge à lèvres une heure après l'application, ni de différence significative de brillance entre la brillance à l'application et celle après une heure. De même, la tenue de la couleur une heure après application est restée identique à celle obtenue lors de l'application.

Un autre test a confirmé que les compositions de base utilisées selon l'invention peuvent être d'excellentes bases de maquillage et permettent une augmentation de la tenue d'un fond de teint dans le temps.

### 3) Test en Flash cabine Soin

Objectif : évaluer les aspects cosmétiques de la composition de base constituée de l'exemple 1 de l'invention, ainsi que l'uniformité d'un fond de teint et d'un rouge à lèvres classiques, appliqués par-dessus cette composition de base, sur 7 femmes.

Le fond de teint ou le rouge à lèvres s'appliquent facilement, l'application est uniforme et les produits appliqués adhèrent bien. Le résultat maquillage est homogène.

## Revendications

1. Produit cosmétique de maquillage et/ou de soin de la peau, des lèvres ou des phanères, contenant une première et une seconde compositions, la première composition consistant en une émulsion E/H comprenant une phase aqueuse dispersée dans une phase huileuse, et contenant au moins une cire et des fibres, et la seconde composition comprenant une phase grasse contenant au moins une huile.

2. Produit selon la revendication 1, **caractérisé en ce que** les fibres ont une longueur allant de 1 µm à 10 mm, de préférence de 0,1 mm à 5 mm.

3. Produit selon la revendication 1 ou 2, **caractérisé en ce que** les fibres ont un section comprise dans un cercle de diamètre allant de 2 nm à 500 µm, de préférence allant de 100 nm à 100 µm.

4. Produit selon l'une des revendications précédentes, **caractérisé en ce que** les fibres sont présentes en une teneur allant de 0,1 à 30 % en poids, de préférence de 1 à 20 % en poids par rapport au poids total de l'émulsion E/H.

5. Produit selon l'une des revendications précédentes, **caractérisé en ce que** les fibres sont choisies parmi les fibres de soie, de coton, de laine, de lin, les fibres de cellulose, de rayonne, de polyamide, de viscose, d'acétate, de poly-p-phénylène téréphtamide, de polymère acrylique, de polyoléfine, de verre, de silice, de carbone, de polytétrafluoroéthylène, de collagène insoluble, de polyesters, de polychlorure de vinyle ou de vinylidène, d'alcool polyvinylique, de polyacrylonitrile, de chitosane, de polyuréthane, de polyéthylène phtalate, les fibres formées d'un mélange de polymères, et leurs mélanges.

6. Produit selon l'une quelconque des revendications précédentes, **caractérisé en ce que** les fibres sont choisies parmi les fibres de polyamide, les fibres de cellulose, les fibres de polyéthylène et leurs mélanges.

7. Produit selon l'une quelconque des revendications précédentes, **caractérisé en ce que** la phase huileuse de l'émulsion E/H se présente sous forme d'une pâte souple à la température ambiante.

8. Produit selon la revendication précédente, **caractérisé en ce que** la pâte souple est obtenue par chauffage des cires et autres corps gras de la phase huileuse, puis malaxage du mélange tout en le refroidissant jusqu'à température ambiante.

9. Produit selon l'une quelconque des revendications précédentes, **caractérisé en ce que** l'émulsion E/H contient un tensioactif émulsionnant choisi dans le groupe comprenant les polydiméthylsiloxanes oxyéthylénés et/ou oxypropylénés, les alkyl-C₁₀-C₂₂-polydiméthylsiloxanes oxyéthylénés et/ou oxypropylénés, les polydimethylsiloxanes oxyéthylénés et/ou oxypropylénés à groupements glucosides, et leurs mélanges.

10. Produit selon la revendication précédente, **caractérisé en ce que** la quantité de tensioactif émulsionnant va de 0,02 à 5 % en poids par rapport au poids total de l'émulsion E/H.

11. Produit selon l'une quelconque des revendications précédentes, **caractérisée en ce que** la cire de l'émulsion E/H est choisie parmi la cire de Carnauba, les cires de polyéthylène ayant une température de fusion commençante supérieure à 50°C, les cires microcristallines ayant une température de fusion commençante supérieure à 50°C, et leurs mélanges.

12. Produit selon l'une quelconque des revendications précédentes, **caractérisé en ce que** la quantité de cire(s) dans l'émulsion E/H va de 4 à 30 % en poids par rapport au poids total de l'émulsion.

13. Produit selon l'une quelconque des revendications précédentes, **caractérisé en ce que** l'émulsion E/H a une quantité de phase huileuse allant de 15 à 70 % en poids par rapport au poids total de l'émulsion.

14. Produit selon l'une quelconque des revendications précédentes, **caractérisé en ce que** la première composition est sous forme de crème.

15. Produit selon l'une quelconque des revendications précédentes, **caractérisé en ce que** la deuxième composition contient une quantité d'huiles représentant au moins 20 % en poids, de préférence au moins 30 % en poids du poids total de ladite composition.

16. Produit selon l'une quelconque des revendications précédentes, **caractérisé en ce que** la première composition contient moins de 0,2 % de pigments tandis que la deuxième composition contient au moins 1 % de pigments.

17. Produit selon l'une des revendications précédentes, **caractérisé en ce qu'**il constitue un fond de teint, un fard à joues ou à paupières, un produit anti-cerne, un blush, un rouge à lèvres, un baume à lèvres, un brillant à lèvres, un crayon à lèvres ou à yeux, un mascara, un eye-liner, un produit de soin de la peau, un produit de maquillage du corps ou de coloration de la peau.

18. Produit selon l'une quelconque des revendications 1 à 16, **caractérisé en ce que** la deuxième composition se présente sous la forme d'un rouge à lèvres en stick, d'un fond de teint coulé, d'un blush coulé ou d'un brillant à lèvres liquide.

19. Kit de maquillage contenant un produit cosmétique de maquillage ou de soin selon l'une des revendications 1 à 16, dans lequel les deux compositions sont conditionnées séparément et sont accompagnées de moyens d'application appropriés.

20. Procédé de maquillage de la peau, des lèvres et/ou des phanères consistant à appliquer sur la peau, les lèvres et/ou les phanères successivement les première et deuxième compositions du produit cosmétique selon l'une des revendications 1 à 16.

21. Utilisation cosmétique d'une composition cosmétique sous forme d'émulsion E/H comprenant au moins une cire et des fibres, pour limiter la migration d'une autre composition comprenant une phase grasse contenant au moins une huile.
